(19) Europäisches Patentamt
European Patent Office
Office européen des brevets

(11) EP 3 272 349 A1

(12) **EUROPEAN PATENT APPLICATION**
published in accordance with Art. 153(4) EPC

(43) Date of publication:
**24.01.2018 Bulletin 2018/04**

(21) Application number: **16765090.2**

(22) Date of filing: **17.03.2016**

(51) Int Cl.:
***A61K 31/522*** *(2006.01)* ***A61P 25/16*** *(2006.01)*
***A61P 25/28*** *(2006.01)*

(86) International application number:
**PCT/JP2016/058592**

(87) International publication number:
**WO 2016/148261 (22.09.2016 Gazette 2016/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **19.03.2015 JP 2015055533**

(71) Applicant: **Kyowa Hakko Kirin Co., Ltd.**
**Tokyo 100-8185 (JP)**

(72) Inventor: **KANDA, Tomoyuki**
**Tokyo 100-8185 (JP)**

(74) Representative: **Vossius & Partner**
**Patentanwälte Rechtsanwälte mbB**
**Siebertstrasse 3**
**81675 München (DE)**

(54) **THERAPEUTIC AND/OR PROPHYLACTIC AGENT FOR LEWY BODY DISEASES**

(57) The present invention provides a therapeutic and/or prophylactic agent and the like, comprising istradefylline as an active ingredient, for Lewy body disease (for example, Parkinson's disease dementia, diffuse Lewy body disease, dementia with Lewy bodies, movement disorder associated with Lewy body disease and the like).

EP 3 272 349 A1

## Description

[Field of the Invention]

The present invention relates to a therapeutic and/or prophylactic agent for Lewy body disease (for example, Parkinson's disease dementia, diffuse Lewy body disease, dementia with Lewy bodies, movement disorder associated with Lewy body disease and the like).

[Background Art]

α-Synuclein is a protein present in large amounts in the intracerebral presynaptic terminal, and a protein involved in synaptic plasticity and neurotransmitter (Journal of Chemical Neuroanatomy, 42, p.242 (2011)). α-Synucleinopathy is a generic term for neurodegenerative diseases characterized by accumulation and formation of aggregation of α-synuclein, and examples thereof comprise Lewy body disease (for example, Parkinson's disease dementia, diffuse Lewy body disease, dementia with Lewy bodies, movement disorder associated with Lewy body disease and the like), multiple system atrophy (for example, olivopontocerebellar atrophy, striatonigral degeneration, Shy-Drager syndrome and the like) and the like.

In Lewy body disease, Lewy bodies containing α-synuclein aggregates as a main component are found in nerve cells and, in multiple system atrophy, α-synuclein-positive inclusion bodies are found in glial cells. Various symptoms such as Parkinsonism, cognitive impairment, autonomic symptom, cerebellar ataxia and the like manifest themselves depending on the level of distribution of these lesions (Parkinsonism and related disorders, 20S1, p.S62 (2014)).

The cognitive impairment in Lewy body diseases is considered to involve dopaminergic and cholinergic hypofunctions in the frontal lobe (Brain, 137, p.2493 (2014); Neurology, 74, p.885 (2010)). The prefrontal cortex of the frontal lobe in the brain is a region responsible for cognitive functions (for example, comprehension, judgment, calculation ability, orientation, executive function) and the like. Neurotransmitters such as dopamine, serotonin, norepinephrine, gamma-aminobutyric acid and the like are involved in the function of prefrontal cortex, and shortage of these substances causes cognitive impairment.

On the other hand, istradefylline (following formula (I)), is known to have an antiparkinson activity (non-patent document 1, patent document 1, patent document 2), a suppressive activity on neurodegeneration (patent document 3), an improving effect on cognitive impairment in Parkinson's disease (non-patent document 2) and the like. Besides, istradefylline is referred in the patent document related to dementia with Lewy bodies (patent document 4).

[Chemical formula 1]

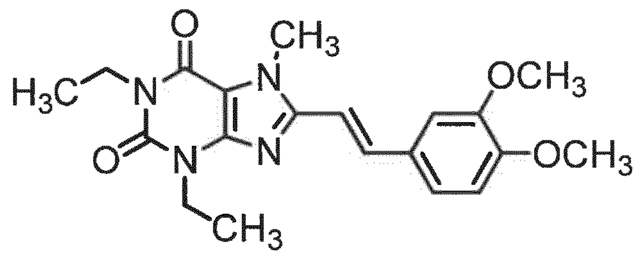

(I)

[PRIOR ART DOCUMENTS]

[PATENT DOCUMENTS]

[Patent Document 1] U.S. Patent No. 5484920
[Patent Document 2] U.S. Patent No. 5587378
[Patent Document 3] WO 99/12546
[Patent Document 4] WO 2010/009557

[NON-PATENT DOCUMENTS]

[Non-patent Document 1] "Annals of Neurology" 1998, vol. 43, p.507-513
[Non-patent Document 2] "Psychopharmacology", 2013, vol. 230, p.345-352

[SUMMARY OF THE INVENTION]

[PROBLEMS TO BE SOLVED BY THE INVENTION]

An object of the present invention is to provide, for example, a therapeutic and/or prophylactic agent for Lewy body disease (for example, Parkinson's disease dementia, diffuse Lewy body disease, dementia with Lewy bodies, movement disorder associated with Lewy body disease and the like).

[MEANS FOR SOLVING THE PROBLEMS]

The present invention relates to the following (1)-(8).

(1) A therapeutic and/or prophylactic agent for Lewy body disease, comprising istradefylline as an active ingredient.
(2) The agent according to (1), wherein the Lewy body disease is Parkinson's disease dementia.
(3) The agent according to (1), wherein the Lewy body disease is diffuse Lewy body disease.
(4) The agent according to (1), wherein the Lewy body disease is dementia with Lewy bodies.
(5) The agent according to (1), wherein the Lewy body disease is movement disorder associated with Lewy body disease.
(6) A method for the treatment and/or prophylaxis of Lewy body disease, comprising a step of administering an effective amount of istradefylline.
(7) Istradefylline for use in the treatment and/or prophylaxis of Lewy body disease.
(8) Use of istradefylline for the manufacture of a therapeutic and/or prophylactic agent for Lewy body disease.

[EFFECT OF THE INVENTION]

According to the present invention, a therapeutic agent and/or prophylactic agent for Lewy body disease (for example, Parkinson's disease dementia, diffuse Lewy body disease, dementia with Lewy bodies, movement disorder associated with Lewy body disease and the like) and the like, which comprises istradefylline as an active ingredient, is provided.

[MODE FOR CARRYING OUT THE INVENTION]

The Lewy body disease in the present invention include, for example, Parkinson's disease dementia, diffuse Lewy body disease, dementia with Lewy bodies, movement disorder associated with Lewy body disease and the like.
Istradefylline, an active ingredient of the present invention may also be used in the form of a salt. The salt encompasses, for example, a pharmaceutically acceptable acid addition salt, a metal salt, an ammonium salt, an organic amine addition salt, an amino acid addition salt and the like. Examples of the pharmaceutically acceptable acid addition salt include inorganic acid salts such as hydrochloride, hydrobromide, nitrate, sulfate, phosphate and the like, organic acid salts such as acetate, oxalate, maleate, fumarate, citrate, benzoate, methanesulfonate and the like, and the like. Examples of the pharmaceutically acceptable metal salt include alkali metal salts such as sodium salt, potassium salt and the like, alkaline earth metal salts such as magnesium salt, calcium salt and the like, aluminum salt, zinc salt and the like. Examples of the pharmaceutically acceptable ammonium salt include salts of ammonium, tetramethylammonium and the like, examples of the pharmaceutically acceptable organic amine addition salt include addition salts with morpholine, piperidine and the like, and examples of the pharmaceutically acceptable amino acid addition salt include addition salts of lysine, glycine, phenylalanine, aspartic acid, glutamic acid and the like.
Istradefylline can be obtained as a commercial product (e.g., NOURIAST (registered trademark)) or by the method described, for example, in US5484920 and the like.
Next, the pharmacological effect of istradefylline is concretely explained by way of Experimental Examples.

[Experimental Example 1] Effect of istradefylline in an α-synucleinopathy model

By reference to articles (Science, 338, p.949 (2012); Behavioral Brain Research, 208, p.274 (2010)), an animal model capable of confirming a therapeutic and/or prophylactic effect on α-synucleinopathy (for example, Lewy body disease, the Lewy body disease includes Parkinson's disease dementia, diffuse Lewy body disease, dementia with Lewy bodies, movement disorder associated with Lewy body disease and the like) is prepared.

An application dose of a solution of α-synuclein (rPeptide, S-100) or NAC61-95 (custom, Sigma) dissolved in phosphate buffered saline (PBS) is injected to the striatum or lateral cerebral ventricle of SLC: ICR male mice to induce cognitive impairment and/or movement disorder. After confirmation of the pathology induction, an application dose of istradefylline is administered to the mice. Pathological improvement of the mice at a few hours after the administration is confirmed by behavioral pharmacological evaluation such as Y-maze, amount of spontaneous motor activity, CATWALK and the like.

[Experimental Example 2] Effect of istradefylline in an α-synucleinopathy model

By reference to articles (Science, 338, p.949 (2012); Behavioral Brain Research, 208, p.274 (2010)), an animal model capable of confirming a therapeutic and/or prophylactic effect on α-synucleinopathy (for example, Lewy body disease, the Lewy body disease includes cognitive impairment in Parkinson's disease, diffuse Lewy body disease, dementia with Lewy bodies, movement disorder associated with Lewy body disease and the like) was prepared as follows.

The cognitive function was evaluated using a spontaneous alternation task. The spontaneous alternation task is known as an evaluation system of cognitive function utilizing properties of an animal to willingly explore a novel environment (Neuroscience and Biobehavioral Reviews 28, p. 497 (2004)). That is, if the animal remembers the arm into which it entered previously in an exploration in a Y-maze apparatus, the behavior of spontaneous entry into a different arm was expressed by an alternation behavior rate, and used as an index of cognitive function. The motor function was evaluated by analyzing gait function under natural walk conditions using a gait function/automatic gait analysis system.

<Preparation of animal model>

NAC61-95 (custom, Sigma) was dissolved in PBS to prepare 4 μg/μL NAC61-95 solution.

Under pentobarbital (Somnopentyl, Kyoritsuseiyaku) (50 mg/kg, i.p.) anesthesia, 5 μL of NAC61-95 solution was injected using a Hamilton syringe (10 μL) equipped with a double needle (27G, needle length 3 mm) into the right lateral cerebral ventricle of Slc:ICR mice (male, Japan SLC) over about 1 minute, and stood for 1 minute to induce cognitive impairment and/or movement disorder.

As for a sham treatment group, under pentobarbital anesthesia, 5 μL of PBS was injected using a Hamilton syringe equipped with a double needle into the right lateral cerebral ventricle of Slc:ICR mice over about 1 minute, and stood for 1 minute.

<Spontaneous alternation task test: evaluation of cognitive function>

A Y-maze apparatus wherein three arms made of a black acrylic wall (height 20 cm, length 25 cm, width 5 cm) are each connected at a 120-degree angle was used.

Mice were brought into the experiment room from the previous day of test for habituation. A mouse was placed at the tip of any of the arms of the Y-maze apparatus and allowed to freely explore in the maze for 7 minutes. Entry of all four limbs of a mouse into one arm was defined as entry into the arm, and the order of entry of the mouse into the arm was recorded. A behavior of successive entry into all three different arms was defined as spontaneous alternation behavior, and the percentage of alternation behavior was calculated by the following calculation formula.

[Formula 1]

$$\text{Alternation behavior (\%)} = \frac{\text{number of spontaneous alternation behaviors}}{(\text{total arm entries}) - 2} \times 100$$

For calculation of the alternation behavior, only the data of an individual with not less than 10 total arm entries was used.

<Gait test: evaluation of motor function>

A gait function/automatic gait analysis system (CatWalk XT, ver.9.1, Noldus) was used. The system consisted of a walkway with pressure-sensitive luminescent glass and a light source, a luminescent ceiling, a highly sensitive high-speed camera and an analysis software, and the gait was analyzed by digitizing the brightness of the light emitted by the pressure-sensitive luminescent glass in response to the pressure. The gait data was automatically recorded and analyzed by the analysis software only when a mouse moved straight forward without stopping in the predetermined area on the walkway.

The mice were brought into the experiment room with light off and habituated for not less than 1 hour. A mouse was placed in the apparatus, and allowed to walk freely until 6 running data were obtained for each individual. The first three running data amenable to analyze were taken up, and their mean was calculated. By reference to an article relating to movement disorder in Parkinson's disease animal model (Journal of Biomedical Science 17, p. 9 (2010)), changes in the maximum brightness ratio (ratio of the duration during which a paw print exhibits maximum brightness to the total duration of paw contact), the print area (a total surface area of the paw print), and gait pattern (a sequence of contact of 4 paws) were used as indices of movement disorder.

<Drug treatment>

At 60 minutes before the test, istradefylline (suspended in 0.5 w/v% aqueous MC solution, and prepared to 0.5 mL per 100 g body weight of a mouse on the administration day) was orally administered at a dose of 1.0 mg/kg, or a vehicle (0.5 w/v% aqueous MC solution) free of the test compound was orally administered at 0.5 mL per 100 g body weight of a mouse on the administration day.

<Results>

Table 1 shows the alternation behavior in the spontaneous alternation task in terms of mean ± standard error.

Table 1

Table 1 Effect of istradefylline on alternation behavior

| group | treatment/ administration | alternation behavior (%) | number of animals (mice) |
|---|---|---|---|
| sham treatment | vehicle/vehicle | 66.9 ± 2.5 | 16 |
| vehicle administration | NAC61-95/vehicle | 58.9 ± 2.7 | 16 |
| istradefylline administration | NAC61-95/istradefylline | 66.4 ± 1.4 | 16 |

The vehicle administration group showed a significantly low alternation behavior as compared to the sham treatment group ($p<0.05$, Student's t-test), and cognitive impairment was found to be induced by NAC61-95 treatment. In contrast, the istradefylline administration group showed a significantly high alternation behavior as compared to the vehicle administration group ($p<0.05$, Aspin-Welch test), and the cognitive impairment by NAC61-95 treatment was found to be improved.

Table 2 shows each gait parameter in the gait test in terms of mean ± standard error.

Table 2

Table 2 Effect of istradefylline on gait parameters

| Group | sham treatment | vehicle administration | istradefylline administration |
|---|---|---|---|
| treatment/ administration | vehicle/vehicle | NAC61-95/vehicle | NAC61-95/istradefylline |
| number of animals (mice) | 16 | 16 | 16 |

(continued)

| Group | | sham treatment | vehicle administration | istradefylline administration |
|---|---|---|---|---|
| maximum brightness ratio | right forepaw | 53.8 ± 2.2 | 54.0 ± 2.3 | 52.5 ± 2.4 |
| | right hindpaw | 62.2 ± 1.6 | 62.5 ± 1.7 | 61.9 ± 2.4 |
| | Left forepaw | 57.3 ± 1.3 | 53.3 ± 2.4 | 51.5 ± 1.8 |
| | Left hindpaw | 64.7 ± 2.1 | 58.4 ± 2.1 | 65.3 ± 1.7 |
| print area | right forepaw | 0.33 ± 0.03 | 0.33 ± 0.02 | 0.33 ± 0.03 |
| | right hindpaw | 0.32 ± 0.03 | 0.31 ± 0.03 | 0.38 ± 0.04 |
| | Left forepaw | 0.34 ± 0.03 | 0.33 ± 0.03 | 0.35 ± 0.02 |
| | Left hindpaw | 0.33 ± 0.03 | 0.35 ± 0.03 | 0.35 ± 0.04 |
| gait pattern (mean) | AA | 0.00 ± 0.00 | 0.35 ± 2.9 | 0.35 ± 2.8 |
| | AB | 91.7 ± 3.1 | 75.4 ± 7.7 | 0.35 ± 7.1 |
| | CA | 0.35 ± 3.0 | 0.35 ± 3.3 | 0.35 ± 3.6 |
| | CB | 0.35 ± 0.4 | 0.35 ± 4.8 | 8.7 ± 4.3 |
| gait pattern (median) | AA | 0.0 (0.0-100.0) | 0.0 (0.0-0.0) | 0.0 (0.0-0.0) |
| | AB | 100.0 (81.0-100.0) | 88.1 (60.2-100.0) | 84.6 (65.2-100.0) |
| | CA | 0.0 (0.0-17.8) | 0.0 (0.0-7.9) | 6.7 (0.0-14.2) |
| | CB | 0.0 (0.0-0.0) | 3.8 (0.0-17.8) | 0.0 (0.0-8.0) |
| AA: right forepaw → right hindpaw → left forepaw → left hindpaw<br>AB: right forepaw → left hindpaw → left forepaw → right hindpaw<br>CA: right forepaw → left forepaw → right hindpaw → left hindpaw<br>CB: right forepaw → left hindpaw → right hindpaw → left forepaw | | | | |

In the vehicle administration group, a significant decrease in the maximum brightness ratio of the left hind paw ($p<0.05$, Student's t-test) and a significant increase in the gait pattern CB ($p<0.01$, Wilcoxon rank sum test) were found as compared to the sham treatment group, and movement disorder was found to be induced by NAC61-95 treatment. In contrast, in the istradefylline administration group, a significant increase in the maximum brightness ratio of the left hindpaw ($p<0.05$, Student's t-test) was found, and the improvement ratio was 111%. The gait pattern CB decreased and the improvement ratio was 100%. From the above, in the istradefylline administration group, the movement disorder induced by NAC61-95 treatment was found to be improved.

From the above-mentioned test, istradefylline was confirmed to have a therapeutic and/or prophylactic effect on $\alpha$-synucleinopathy (for example, Lewy body disease (the Lewy body disease includes Parkinson's disease dementia, diffuse Lewy body disease, dementia with Lewy bodies, movement disorder associated with Lewy body disease and the like) and the like).

While istradefylline can be directly administered singly, it is generally desirably provided as various pharmaceutical preparations. Such pharmaceutical preparations are used for animals or humans.

The pharmaceutical preparation of the present invention can contain istradefylline or a pharmaceutically acceptable salt thereof singly as an active ingredient or as a mixture with any other active ingredients. In addition, such pharmaceutical preparations are produced by mixing the active ingredient with one or more kinds of pharmaceutically acceptable carriers (for example, diluent, solvent, excipient and the like), and according to any method well known in the technical field of drug formulation study.

As an administration route, a route most effective for the treatment is desirably used, which may be oral or parenteral, for example, intravenous, transdermal and the like.

Examples of the administration form include tablet, injection, external preparation and the like.

A form suitable for oral administration, for example, tablet and the like, can be produced using an excipient such as lactose and the like, a disintegrant such as starch and the like, a lubricant such as magnesium stearate and the like, a binder such as hydroxypropylcellulose and the like, and the like.

A form suitable for parenteral administration, for example, injection and the like can be produced using a diluent or solvent such as a salt solution, a glucose solution, a mixture of salt water and a glucose solution and the like, and the like.

While a dosage form suitable for external preparation is not particularly limited, for example, ointment, cream, liniment, lotion, cataplasm, plaster, tape and the like can be mentioned. For example, ointment, cream and the like can be produced by, for example, dissolving or mixing and dispersing the active ingredient in a base such as white petrolatum and the like.

The dose and administration frequency of istradefylline or a pharmaceutically acceptable salt thereof vary depending on the administration form, age and body weight of patients, nature or severity of the symptom to be treated and the like. In the case of oral route, 0.01-1000 mg, preferably 0.05-100 mg, is generally administered to an adult once to several times per day. In the case of parenteral administration such as intravenous administration and the like, 0.001-1000 mg, preferably 0.01-100 mg, is generally administered to an adult once to several times per day. In the case of transdermal administration, an external preparation containing 0.001-10% of istradefylline or a pharmaceutically acceptable salt thereof is generally administered by applying once to several times per day. However, such dose and administration frequency vary depending on the aforementioned various conditions.

[EXAMPLES]

The present invention will be described in more detail by way of Examples, but the present invention is not limited by the following Examples.

[Example 1]

A tablet having the following composition is prepared by a conventional method. Istradefylline (40 g), lactose (286.8 g) and potato starch (60 g) are mixed, and 10% aqueous solution (120 g) of hydroxypropylcellulose is added thereto. The mixture is kneaded, granulated, dried, and sieved to give granules for tableting by a conventional method. The granules are mixed with magnesium stearate (1.2 g) and the mixture is tableted by a tableting machine with a 8 mm punch (manufactured by Kikusui, RT-15) to give tablets (containing 20 mg of active ingredient per tablet).

Table 3

| Formulation | |
|---|---|
| istradefylline | 20 mg |
| lactose | 143.4 mg |
| potato starch | 30 mg |
| hydroxypropylcellulose | 6 mg |
| magnesium stearate | 0.6 mg |
| | 200 mg |

[Example 2]

An injection having the following composition is prepared by a conventional method. Istradefylline (1 g) is mixed with injectable distilled water, pH is adjusted to 7 by adding hydrochloric acid and aqueous sodium hydroxide solution, and injectable distilled water is added to make the total amount of 1000 mL. The obtained mixture is aseptically filled in a glass vial by 2 mL to give injections (containing 2 mg of active ingredient per vial).

Table 4

| Formulation | |
|---|---|
| istradefylline | 2 mg |
| hydrochloric acid | q.s. |
| aqueous sodium hydroxide solution | q.s. |
| injectable distilled water | q.s. |
| | 2.00 mL |

[INDUSTRIAL APPLICABILITY]

The present invention can be used for treating and/or preventing, for example Lewy body disease (for example, Parkinson's disease dementia, diffuse Lewy body disease, dementia with Lewy bodies, movement disorder associated with Lewy body disease and the like).

**Claims**

1. A therapeutic and/or prophylactic agent for Lewy body disease, comprising istradefylline as an active ingredient.

2. The agent according to claim 1, wherein the Lewy body disease is Parkinson's disease dementia.

3. The agent according to claim 1, wherein the Lewy body disease is diffuse Lewy body disease.

4. The agent according to claim 1, wherein the Lewy body disease is dementia with Lewy bodies.

5. The agent according to claim 1, wherein the Lewy body disease is movement disorder associated with Lewy body disease.

## INTERNATIONAL SEARCH REPORT

International application No.
PCT/JP2016/058592

A. CLASSIFICATION OF SUBJECT MATTER
*A61K31/522*(2006.01)i, *A61P25/16*(2006.01)i, *A61P25/28*(2006.01)i

According to International Patent Classification (IPC) or to both national classification and IPC

B. FIELDS SEARCHED

Minimum documentation searched (classification system followed by classification symbols)
A61K31/522, A61P25/16, A61P25/28

Documentation searched other than minimum documentation to the extent that such documents are included in the fields searched
Jitsuyo Shinan Koho 1922-1996 Jitsuyo Shinan Toroku Koho 1996-2016
Kokai Jitsuyo Shinan Koho 1971-2016 Toroku Jitsuyo Shinan Koho 1994-2016

Electronic data base consulted during the international search (name of data base and, where practicable, search terms used)
CAplus/REGISTRY/MEDLINE/EMBASE/BIOSIS(STN), JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| X<br>Y | JP 6-211856 A (Kyowa Hakko Kogyo Co., Ltd.), 02 August 1994 (02.08.1994), claims; paragraph [0002]; paragraph [0059], compound 2; test example 3 & EP 590919 A1 claims; page 3; page 11, compound 2; experimental example 3 & US 5484920 A & DE 69327438 D | 1,5<br>2-4 |
| Y | Genta ITO et al., "Lewy Shotai to Shinkei Saibo Hensei Kijo", Japanese Journal of Clinical Psychiatry, 2004, vol.33, no.1, pages 13 to 19, ISSN 0300-032X, paragraphs of '1.Introduction', '2.Lewy Shotai', '3.α-synuclein to Shinkei Saibo Hensei' | 2-4 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
06 April 2016 (06.04.16)

Date of mailing of the international search report
19 April 2016 (19.04.16)

Name and mailing address of the ISA/
Japan Patent Office
3-4-3,Kasumigaseki,Chiyoda-ku,
Tokyo 100-8915,Japan

Authorized officer

Telephone No.

Form PCT/ISA/210 (second sheet) (January 2015)

**INTERNATIONAL SEARCH REPORT**

International application No.
PCT/JP2016/058592

C (Continuation). DOCUMENTS CONSIDERED TO BE RELEVANT

| Category* | Citation of document, with indication, where appropriate, of the relevant passages | Relevant to claim No. |
|---|---|---|
| A | WO 2010/009557 A1 (SANOMUNE INC.), 28 January 2010 (28.01.2010), claims; [00101]; examples & US 2011/0150781 A1 & EP 2320939 A1 | 1-5 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5484920 A
- US 5587378 A
- WO 9912546 A
- WO 2010009557 A


### Non-patent literature cited in the description


- *Journal of Chemical Neuroanatomy,* 2011, vol. 42, 242
- *Parkinsonism and related disorders,* 2014, vol. 20S1, S62
- *Brain,* 2014, vol. 137, 2493
- *Neurology,* 2010, vol. 74, 885
- *Annals of Neurology,* 1998, vol. 43, 507-513
- *Psychopharmacology,* 2013, vol. 230, 345-352
- *Science,* 2012, vol. 338, 949
- *Behavioral Brain Research,* 2010, vol. 208, 274
- *Neuroscience and Biobehavioral Reviews,* 2004, vol. 28, 497
- *Journal of Biomedical Science,* 2010, vol. 17, 9